# EUROPEAN PATENT SPECIFICATION

(11) **EP 3 193 841 B1**
(45) Date of publication and mention of the grant of the patent: **18.09.2019**
(21) Application number: 15777751.7
(22) Date of filing: 14.08.2015
(51) Int. Cl.: A61K 9/16, A61K 9/50, A61K 31/726, A61P 13/10, A61K 41/00, A61K 9/00

(54) **DEVELOPMENT OF MAGNETIC-MICRO PARTICLES THAT PROVIDE CONTROLLED GLYCOSAMINOGLYCAN (GAG) RELEASE AND THE INTRAVESICAL USAGE OF IT IN INTERSTITIAL CYSTITIS**
ENTWICKLUNG VON MAGNETISCHEN MIKROPARTIKELN ZUR GESTEUERTEN FREISETZUNG VON GLYKOSAMINOGLYKAN (GAG) UND INTRAVESIKALE VERWENDUNG DAVON BEI INTERSTITIELLER ZYSTITIS
DÉVELOPPEMENT DE MICROPARTICULES MAGNÉTIQUES OFFRANT UNE LIBÉRATION CONTRÔLÉE DE GLYCOSAMINOGLYCANE (GAG) ET SON UTILISATION INTRAVÉSICALE DANS DES CAS DE CYSTITE INTERSTITIELLE

(30) Priority: 17.09.2014 TR 201410933
(43) Date of publication of application: 26.07.2017
(73) Proprietor: Erol, Bülent, Istanbul (TR)
(72) Inventor: Erol, Bülent, Istanbul (TR)
(74) Representative: Yamankaradeniz, Kemal
(86) International application number: PCT/TR2015/050070
(87) International publication number: WO 2016/043680

(56) References cited:
- US-A- 5 427 767
- US-A- 5 591 724
- GUHASARKAR S ET AL: "Intravesical drug delivery: Challenges, current status, opportunities and novel strategies", JOURNAL OF CONTROLLED RELEASE, ELSEVIER, AMSTERDAM, NL, vol. 148, no. 2, 1 December 2010 (2010-12-01), pages 147-159, XP027509905, ISSN: 0168-3659, DOI: 10.1016/J.JCONREL.2010.08.031 [retrieved on 2010-09-08]
- LEAKAKOS TINA ET AL: "Intravesical administration of doxorubicin to swine bladder using magnetically targeted carriers.", CANCER CHEMOTHERAPY AND PHARMACOLOGY, vol. 51, no. 6, June 2003 (2003-06), pages 445-450, XP002751868, ISSN: 0344-5704

## Description

### Technical Field

The application field of the invention is Urology-Neurourology under Surgical Medical Sciences title.

The invention especially relates to interstitial cystitis minimal invasive treatment field. With the present invention, the damaged glycosaminoglycan layer in bladder due to cystitis is constantly repaired.

### State of the Art

Interstitial Cystitis is a disease which does not have a certain treatment yet. The most important reason for this is the defect of the Glycosaminoglycan layer that covers the inner surface of the bladder and the degeneration created on the inner surface of the bladder by the urine with toxic effect. At the present time although the most effective treatment seems to be intravesical drug use alongside oral treatment, the benefit of this treatment is limited and it has two disadvantages. The first one is the necessity of application in the bladder via probe or urethral catheter 6 or 8 times in every other week, the second one is the short effectiveness of the treatment. The most important reason for this is that the IC patients cannot hold their urine for long times, the drug does not contact the bladder enough or it does not reach the sufficient level on the bladder tissue. Therefore the GAG layer repair remains temporary and insufficient.

To this end, nanotechnology is started to be used in in vitro intravesical applications. The most important advantage of this new technique is that it makes the penetration to the bladder tissue easier for the drug to be given to the bladder thanks to its drug carrying system function. However, the carriage and penetration of the drug to the bladder are not single handedly sufficient to make the treatment effectiveness last long.

There are a few intravesical agents which are used in routine clinical applications for interstitial cystitis. When these agents are examined in the light of literature, Dimethyl sulfoxide (DMSO) is the only agent among these drugs that has FDA approval. It has been shown that the intravesical application of DMSO provides objective and subjective benefits to the patients and is more effective compared to placebo. It is applied weekly for 6 weeks of cure. There are studies showing that intravesical glycosaminoglycans such as heparin, hyaluronic acid, chondroitin sulfate, pentosan polysulfate are beneficial. The usage of these agents are limited by the lack of prospective randomized studies, the insufficient effectiveness of these drugs and the abundance of their side effects.

There is limited information in the literature on interstitial cystitis model and nanotechnology. When we look at these rare studies, it is seen that different types of nanoparticles have different effects on the bladder, indeed. The studies are at in vitro level. Seven types of nanoparticles that may affect the bladder are defined as; Mucoadhesive (Chitosan, synthetic polymers), Dendrimers, Protein (gel nanoparticle), Lipid (Liposome), magnetic, Gold Nanoshell, in situ gel systems.

Chitosan which is one of the mucoadhesive nanoparticles is used as a drug (mitomycin C) carrier system in bladder cancer due to its bladder urothelium permeability increasing function.

Protein Nanoparticles (gel, etc.) are used in bladder cancer as paclitaxel carriers.

It is shown that in the interstitial cystitis model of liposome (one of the lipid nanoparticles) which is made by using Protamine Sulphate, it protects urothelium from irritant penetration thanks to the lipid biofilm layer it creates on the bladder surface.

Lastly, the Magnetic Nanoparticles are mostly used in diagnostic MR and in a study it is stated that they played a role as doxorubicin carriers in the bladder.

During the research made in the literature, some applications on the subject were encountered. One of these is the patent application publication No. TR201103387 titled "Pharmaceutical composition comprising quinuclidin-3'-yl 1-phenyl-1,2,3,4,-tetrahydroisoquinoline-2-carboxylate for treatment of interstitial cystitis and/or abacterial prostatitis". The summary of the invention is as follows: 'A depressant of capsaicin-sensitive sensory nerve, containing quinuclidine-3'-yl 1-phenyl-1,2,3,4-tetra-hydroisoquinoline-2-carboxylate or a salt there of as an active ingredient, specifically a therapeutic drug of interstitial cystitis, hypersensitive disorder of the lower urinary tract, and/or abacterial prostatitis.'

Another similar one is the patent application publication No. TR 201203857 titled 'PDE 4 inhibitors for the treatment of interstitial cystitis'. The summary of the invention is as follows: This invention relates to low molecular weight sodium hyaluronate usage between 120 kdalton and 200 kdalton range, in 50 to 180 mg/50ml solution, by creating a temporary layer on the bladder wall in the treatment of interstitial cystitis, recurrent bacterial cystitis and radiation cystitis.

Another patent application with publication No. US5591724 (A**)** titled "Method for treating the urinary bladder and associated structures using hyaluronic acid" relates to a method of treating interstitial cystitis comprising contacting the internal bladder and associated structures in a mammal having interstitial cystitis with a solution containing hyaluronic acid having a molecular weight of not less than approximately 2x105 Daltons in a concentration effective to treat the interstitial cystitis. This application discloses the treatment of interstitial cystitis by instillation of a solution of heparin into the bladder, however it does not disclose the use of magnetic particles.

Another patent application with publication No. US5427767 (A**)** titled "Nanocrystalline magnetic iron oxide particles-method for preparation and use in medical diagnostics and therapy" relates to nanocrystalline magnetic particles consisting of magnetic iron oxide core of Fe3O4, gamma-Fe2O3 or mixtures thereof and an envelope chemisorbed to said core, the method for preparation of these particles as well as the use thereof in medical diagnostics and/or therapy. The magnetic particles, according to the invention, are characterized by composition of the coating material of natural or synthetic glycosaminoglycans and/or their derivatives with molecular weights of 500 Da to 250,000 Da, if necessary, covalently cross-linked with appropriate cross-linking agents and/or modified by specific additives. This application discloses magnetic nanoparticles coated with chondroitin sulfate, dermatan sulfate, heparan sulfate, heparin or heparinoids. The particles are intended for use in diagnostics and therapy. However, this application does not disclose the treatment of interstitial cystitis.

Another similar one is an article titled "Intravesical drug delivery: Challenges, current status, opportunities and novel strategies", JOURNAL OF CONTROLLED RELEASE, ELSEVIER, AMSTERDAM, NL, vol. 148, no. 2,1 December 2010 (2010-12-01), pages 147-159, XP027509905, ISSN: 0168-3659, DOI: 10.1016/J.JCONREL.2010.08.031 [retrieved on 2010-09-08]. This scientific review article on intravesical drug delivery and discloses that glycosaminoglycans such as pentosane sulfate may be used for treatment of interstitial cystitis and further discloses various strategies for prolonging residence time in the bladder, including magnetic nanoparticles. However, this article does not disclose the combination of glycosaminoglycans with magnetic nanoparticles.

As a result, an improvement in the technical field on the treatment method of IC disease has become a necessity due to the insufficiencies of the current solutions on the subject because of the problems mentioned above.

### Objective of the Invention

The present invention is based on the development of magnetic-micro particles that meet the above mentioned necessities, removes all disadvantages and bring some additional advantages, provide controlled Glycosaminoglycan (GAG) release; and the usage of intravesical for interstitial cystitis.

The main objective of the invention is to provide the drugs which are intravesically applied, to reach the bladder tissue easier thanks to micro particles obtained through nanotechnology. Also, by means of the interaction between the Magnetic micro particle which makes GAG release and magnetic tape that is placed in suprapubic area, the drug is intended to stay in the bladder for a long time.

Another objective of the invention is to prevent the drug to be thrown out of the bladder in a short time in interstitial cystitis patients and, to enable the repairing of long term GAG defect as a result of bladder being exposed to more drugs thanks to this carrier system that uses Bioadhesion.

A magnetic tape placed in the suprapubic area and a release mechanism comprising of the drug, which provides a grip in the bladder, has magnetic feature and presents slow release, are used in the invention. This mechanism can be used in all lumen organs and in case any drug is desired to be released for a long time; in all lumen organs, all urinary system in urology, intrauterine applications in gynecology, lumen organs like stomach-intestines in gastroenterology.

The invention therefore relates to magnetic microparticles coated with glycosaminoglycan for use in the repair of damaged glycosaminoglycan layer in the bladder due to interstitial cystitis.

The invention also relates to a kit for use in the repair of glycosaminoglycan layer which is damaged in the bladder due to interstitial cystitis, characterized in that it comprises said magnetic microparticles coated with glycosaminoglycan, and subcutaneous magnetic tape that interacts with magnetic microparticle which releases glycosaminoglycan, whereby said subcutaneous magnetic tape is to be placed in the suprapubic area in order for said glycosaminoglycan to get a grip in the bladder and make it stay in the bladder for a long time.

Structural and characteristic features and all advantages of the invention will be understood more clearly through the figures and detailed descriptions made with reference to these figures below and therefore the evaluation needs to be carried out by considering these figures and detailed descriptions.

### Figures that Help the Invention to be Understood Better

**Figure 1** is the view of Glycosaminoglycan (GAG) layer defect in interstitial cystitis.
**Figure 2** is the view showing the drug, in the injector, which is prepared with nanotechnology, has magnetic feature and can make slow release.
**Figure 3** is the view of subcutaneous magnetic tape that is placed in the suprapubic area to hold the drug with magnetic feature in the bladder.
**Figure 4** is the view showing the drug spreading on the bladder inner surface in the style of a gel.

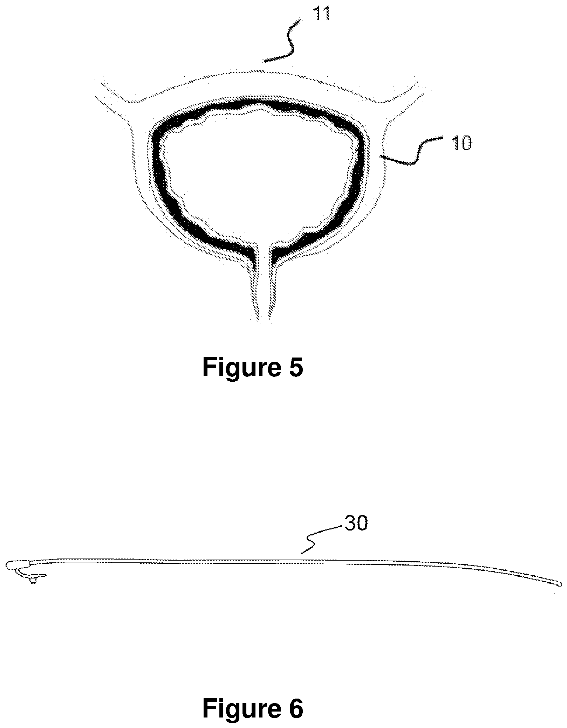

**Figure 5** is the view of a healthy bladder.
**Figure 6** is the view of a urethral catheter.

The drawings should not necessarily be scaled and the details that are not necessary to understand the invention might have been neglected. Other than this, the elements that are at least identical to a great extent or that are identical to a great extent at least in terms of their functions are shown with the same numbers.

### Explanation of Part References

**1.** Kit
**10.** Bladder
**11.** Suprapubic area
**20.** Magnetic tape
**21.** Suture
**30.** Urethral catheter
**40.** Syringe

### Detailed Explanation of the Invention

In this detailed explanation, the preferred embodiments of the structuring of development of magnetic-micro particles that provide controlled Glycosaminoglycan (GAG) release and the intravesical usage of it in Interstitial cystitis method according to the invention are described only for the subject to be understood better without any limiting effect.

First of all, magnetic microparticles coated with GAG are prepared to be used in the invention.

### Preparation Method of Molecule with Nanotechnology

**Intravesical Drug:** The content of the GAG which is the main substance will be coated on the microparticles and prepared in nanotechnology laboratory (it will transform into gel form in the bladder).

**Intravesical drug preparation method:** Magnetic microparticles that include 5mg/kg GAG will be introduced into the bladder with a layer by layer method.

### Synthesis of Magnetic Microparticles

In the method of the present invention, the magnetic microparticles will be synthesized with reflux method. A certain amount of Fe³⁺ salt (for example FeCl₃.4H₂O) will be dissolved in 50 ml water in a 3 necked flask, then reflux is applied for 2 hours at 100 °C on heating mantle. The iron solution which is brown in color before the reflux procedure gets a black color at the end of 2 hours and solid particle formation at the bottom of the flask is observed. Then the solid product in the 3 necked flask is separated from the solution using magnetic separation method and is dried for 2 hours at 60 °C after being washed twice with water and once with ethyl alcohol. In order for the surface of the synthesized Fe₃O₄ particle not to be oxidized due to the oxygen in the air and form a Fe₂O₃ layer on the surface, N₂ gas should be passed through the reaction environment throughout the synthesis. The main advantage of this method is that it is a green synthesis method which means that the chemical material used for the synthesis is only Fe³⁺ salt and there is no need for any base solution in order for the precipitation to happen. The sizes of the magnetic particles to be synthesized are aimed to be 1-5 µm. The synthesized magnetic particles will be characterized using FTIR, XRD and TEM methods of analysis.

### Covering the Surfaces of the Magnetic Micro Particles with GAG

First, the surfaces of the synthesized magnetic particles will be modified with polyacrylic acid. For this procedure; magnetic particles will be added to the polyacrylic acid solution which will be solved in water and after approximately 6 hours of mixing at 50°C it will be separated using a magnet and washed. Acquired acid functional particles will be added to first collagen solution (1% water solution) and then GAG solution (1% water solution) using layer by layer method and will be mixed for 3 hours in each solution. After that, after will be washed with water each time, using separation by magnet. Magnetic parts will be covered approximately with 100 layers and then will be dried in room temperature. The release feature of the covered polymer layers will be gravimetrically applied to the acquired particles developing solutions similar to time based urine solution. After determining the most appropriate number of layers *in vivo* studies will be made.

Said invention consists of two fixed parts:
1. GAG with magnetic micro particles (Drug)
2. A **KIT** consisted of a combination of Magnetic Tapes (20)

Development of magnetic microparticles that can release drug (GAG) in a controlled fashion and holding this molecule in the bladder (10) are provided by the invention. Holding the GAG coated magnetic particles in the bladder (10) is provided by the magnetic interaction between magnetic tapes (20) that are placed in the suprapubic area (11) and the magnetic particles.

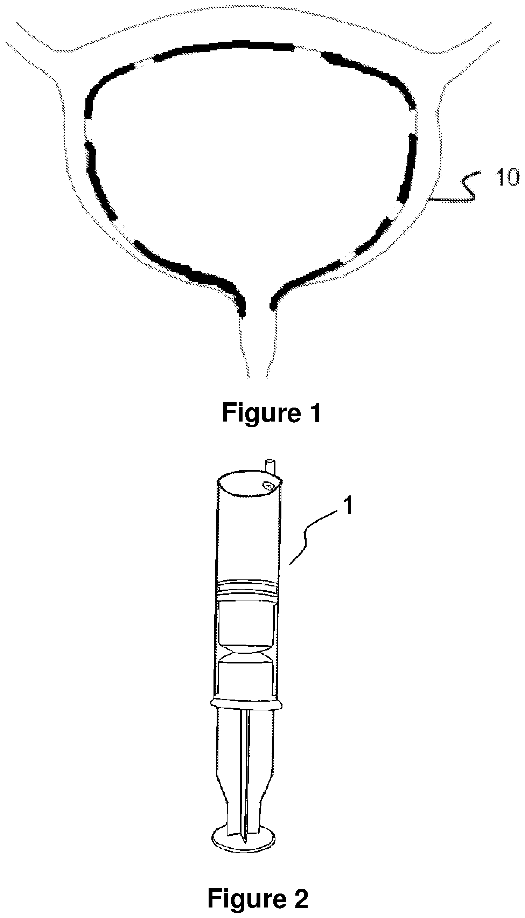

A view of Glycosaminoglycan (GAG) layer defect in interstitial cystitis is illustrated in Figure 1. When interstitial cystitis is formed, defect is seen in (GAG) layer of the bladder (10). In that case, the kit (GAG with magnetic micro particles) application that might be positioned in the place of the layer that disappeared is made as follows;

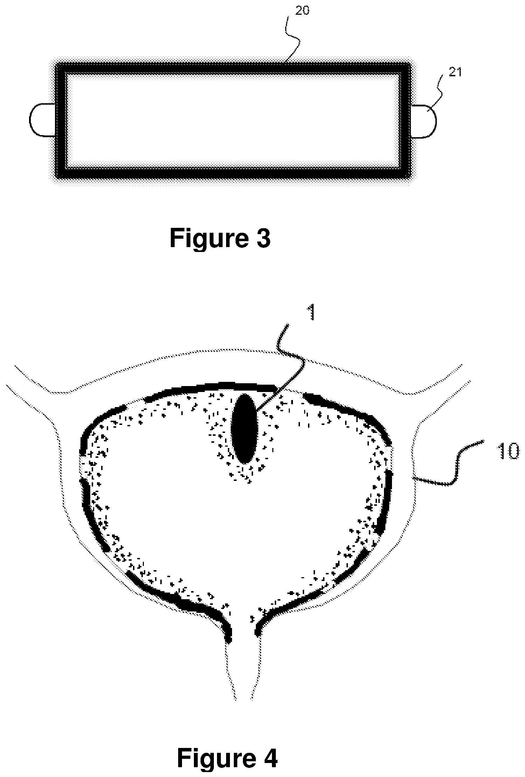

In Figure 3 there is the view of subcutaneous magnetic tape (20) that is placed in suprapubic area (11) for the GAG which is coated on magnetic featured microparticles to get a grip in the bladder (10). In Figure 4 there is the view of the kit (1) spreading in gel style to the inner surface of the bladder (10). First, GAG coated magnetic microparticles is prepared as mentioned above in detail. Then magnetic tape (20) is placed in suprapubic area (11). The reason for the placement of magnetic band (20) in suprapubic area (11) is to ensure the GAG with magnetic microparticles get a grip in the bladder (10) and spread homogeneously. After the magnetic tape (20) is placed in the suprapubic area (11), it is fixed via suture (21). Then, via syringe, the prepared GAG with magnetic microparticles is introduced into the bladder (10) with the help of 10F urethral catheter (30). When the GAG with magnetic microparticles is injected in the bladder (10); it interacts with the magnetic tape (20) that is placed in the suprapubic area (11) and spreads in the bladder (10) providing it to hold for a long time in the bladder (10). Therefore the interstitial cystitis patients are aimed to need the treatment in less frequent periods.

Thanks to this kit (1) which can stay in the bladder (10) for a long time and release slowly, the treatment method that does not require frequent intravesical application is defined in interstitial cystitis (IC) patients.

## Claims

1. Magnetic microparticles coated with glycosaminoglycan for use in the repair of damaged glycosaminoglycan layer in the bladder due to interstitial cystitis.

2. Magnetic microparticles for use according to Claim 1, **characterized in that** said magnetic micro particles are synthesized with the reflux method.

3. Magnetic microparticles for use according to Claim 2, **characterized in that** said reflux method comprises process step of reflux application for 2 hours at 100 °C on heating mantle after getting Fe³⁺ salt and dissolving it in a flask with the help of water.

4. Magnetic microparticles for use according to Claim 3, **characterized in that** the iron solution which is brown in color before the reflux procedure gets a black color at the end of 2 hours and solid particle formation is observed at the bottom of the flask.

5. Magnetic microparticles for use according to Claim 4, **characterized in that** the solid product in the flask from is separated by using magnetic separation method.

6. Magnetic microparticles for use according to Claim 5, **characterized in that** the product separated from the solution by using magnetic separation method is dried for 2 hours at 60 °C after being washed with water and ethyl alcohol.

7. Magnetic microparticles for use according to Claim 6, **characterized in that** N₂ gas is passed through the reaction environment throughout the synthesis in order for the surface of the synthesized Fe₃O₄ particle not to be oxidized due to the oxygen in the air and form a Fe₂O₃ layer on the surface.

8. Magnetic microparticles for use according to Claim 7, **characterized in that** the sizes of synthesized magnetic parts are 1-5 µm.

9. Magnetic microparticles for use according to Claim 8, **characterized in that** the surface of the synthesized magnetic particles is modified with polyacrylic acid first.

10. Magnetic microparticles for use according to Claim 9, **characterized in that** said modification procedure with polyacrylic acid comprises of the process steps of;
• adding magnetic particles to polyacrylic acid solution which is dissolved in water and mixing them,
• after mixing, separating them via magnet and washing.

11. Magnetic microparticles for use according to Claim 10, **characterized in that** by using layer by layer method the acquired acid functional particles comprise of the process steps of;
• adding them first to collagen solution,
• then to glycosaminoglycan solution and mixing them for 3 hours in each solution.

12. Magnetic microparticles for use according to Claim 11, **characterized in that** they are washed with water each time, by using separation by magnet method after said mixing procedure.

13. A kit (1) for use in the repair of glycosaminoglycan layer which is damaged in the bladder due to interstitial cystitis, **characterized in that** it comprises
• magnetic microparticles coated with glycosaminoglycan mentioned in Claim 1 or 12,
• subcutaneous magnetic tape (20) that interacts with magnetic microparticle which releases glycosaminoglycan,
whereby said subcutaneous magnetic tape is to be placed in the suprapubic area in order for said glycosaminoglycan to get a grip in the bladder (10) and make it stay in the bladder (10) for a long time.

14. The kit (1) for use according to Claim 13, **characterized in that** said magnetic tape (20) is to be fixed to the suprapubic area via suture (21).

15. The kit (1) for use according to Claim 13, **characterized in that** said magnetic microparticles coated with glycosaminoglycan are to be introduced into the bladder (10) via syringe (40).

## Patentansprüche

1. Magnetische Mikropartikel, beschichtet mit Glykosaminoglykan zur Verwendung bei der Reparatur von geschädigter Glykosaminoglykanschicht in der Blase aufgrund von interstitieller Zystitis.

2. Magnetische Mikropartikel zur Verwendung nach Anspruch 1, **dadurch gekennzeichnet, dass** die magnetischen Mikropartikel mit dem Rückflussverfahren synthetisiert sind.

3. Magnetische Mikropartikel zur Verwendung nach Anspruch 2, **dadurch gekennzeichnet, dass** das Rückflussverfahren den Prozessschritt der Rückflussanwendung für 2 Stunden bei 100 °C auf Heizmantel umfasst, nachdem Fe³⁺ Salz hinzugefügt wurde und in einem Kolben mithilfe von Wasser gelöst wurde.

4. Magnetische Mikropartikel zur Verwendung nach Anspruch 3, **dadurch gekennzeichnet, dass** die Eisenlösung, die vor der Rückflussprozedur die Farbe Braun aufweist, am Ende der 2 Stunden schwarz wird, und die Bildung von festen Partikeln am Kolbenboden beobachtet wird.

5. Magnetische Mikropartikel zur Verwendung nach Anspruch 4, **dadurch gekennzeichnet, dass** das feste Produkt aus dem Kolben unter Verwendung von magnetischem Trennverfahren getrennt wird.

6. Magnetische Mikropartikel zur Verwendung nach Anspruch 5, **dadurch gekennzeichnet, dass** das durch magnetisches Trennverfahren aus der Lösung getrennte Produkt für 2 Stunden bei 60 °C getrocknet wird, nachdem es mit Wasser und Ethylalkohol gewaschen wurde.

7. Magnetische Mikropartikel zur Verwendung nach Anspruch 6, **dadurch gekennzeichnet, dass** N₂-Gas die Reaktionsumgebung während der Synthese durchströmt, damit die Oberfläche des synthetisierten Fe₃O₄ -Partikels nicht aufgrund des Sauerstoffs in der Luft oxidiert und sich eine Fe₂O₃ -Schicht auf der Oberfläche bildet.

8. Magnetische Mikropartikel zur Verwendung nach Anspruch 7, **dadurch gekennzeichnet, dass** die Größe der synthetisierten magnetischen Partikel 1-5 µm beträgt.

9. Magnetische Mikropartikel zur Verwendung nach Anspruch 8, **dadurch gekennzeichnet, dass** die Oberfläche der synthetisierten magnetischen Partikel zuerst mit Polyakrylsäure modifiziert wird.

10. Magnetische Mikropartikel zur Verwendung nach Anspruch 9, **dadurch gekennzeichnet, dass** die Modifikationsprozedur mit Polyakrylsäure die folgenden Prozessschritte umfasst:
• Zufügen von magnetischen Partikeln zur Polyakylsäurelösung, die in Wasser gelöst ist und Mischen,
• Nach dem Mischen, Trennen durch Magnet und Waschen.

11. Magnetische Mikropartikel zur Verwendung nach Anspruch 10, **dadurch gekennzeichnet, dass** durch die Verwendung des Layer-by-Layer-Verfahrens an den erworbenen säurefunktionellen Partikeln die folgenden Prozessschritte umfasst:
• Zuerst Zufügen zur Kollagenlösung,
• Dann zur Glykosaminoglykanlösung und Mischen für 3 Stunden in jeder Lösung.

12. Magnetische Mikropartikel zur Verwendung nach Anspruch 11, **dadurch gekennzeichnet, dass** sie jedes Mal mit Wasser gewaschen werden, durch Verwendung der Trennung durch Magnetverfahren nach der Mischprozedur.

13. Kit (1) zur Verwendung bei der Reparatur von Glykosaminoglykanschicht, die geschädigt ist, in der Blase aufgrund von interstitieller Zystitis, **dadurch gekennzeichnet, dass** es Folgendes umfasst
• magnetische Mikropartikel, beschichtet mit Glykosaminoglykan, die in Anspruch 1 oder 12 erwähnt werden,
• subkutanes magnetisches Band (20), das mit den magnetischen Mikropartikeln zusammenwirkt, die Glykosaminoglykan freisetzen,
wodurch das subkutane magnetische Band im suprapubischen Bereich platziert wird, damit das Glykosaminoglykan in der Blase (10) zurückgehalten wird und es in der Blase (10) über eine lange Zeit bleibt.

14. Kit (1) zur Verwendung nach Anspruch 13, **dadurch gekennzeichnet, dass** das magnetische Band (20) auf dem suprapubischen Bereich durch eine Naht (21) fixiert wird.

15. Kit (1) zur Verwendung nach Anspruch 13, **dadurch gekennzeichnet, dass** die magnetischen Mikropartikel, beschichtet mit Glykosaminoglykan in die Blase (10) durch eine Nadel (40) eingeführt wird.

## Revendications

1. Microparticules magnétiques revêtues de glycosaminoglycane destinées à être utilisées dans la réparation d'une couche endommagée de glycosaminoglycane située dans la vessie à cause d'une cystite interstitielle.

2. Microparticules magnétiques destinées à être utilisées selon la revendication 1, **caractérisées en ce que** lesdites microparticules magnétiques sont synthétisées au moyen du procédé de reflux.

3. Microparticules magnétiques destinées à être utilisées selon la revendication 2, **caractérisées en ce que** ledit procédé de reflux comprend une étape de processus d'application de reflux pendant 2 heures à 100 °C sur un chauffe-ballon après avoir obtenu un sel de Fe³⁺ et sa dissolution dans un ballon à l'aide d'eau.

4. Microparticules magnétiques destinées à être utilisées selon la revendication 3, **caractérisées en ce que** la solution de fer qui est de couleur brune avant la procédure de reflux devient de couleur noire après 2 heures et la formation de particules solides est observée au fond du ballon.

5. Microparticules magnétiques destinées à être utilisées selon la revendication 4, **caractérisées en ce que** le produit solide dans le ballon est séparé au moyen d'un procédé de séparation magnétique.

6. Microparticules magnétiques destinées à être utilisées selon la revendication 5, **caractérisées en ce que** le produit séparé de la solution au moyen d'un procédé de séparation magnétique est séché pendant 2 heures à 60 °C après avoir été lavé avec de l'eau et de l'alcool éthylique.

7. Microparticules magnétiques destinées à être utilisées selon la revendication 6, **caractérisées en ce que** du N₂ gazeux est passé à travers l'environnement réactionnel par l'intermédiaire de la synthèse de façon à ce que la surface de la particule de Fe₃O₄ synthétisée ne soit pas oxydée à cause de l'oxygène présent dans l'air et forme une couche de Fe₂O₃ sur la surface.

8. Microparticules magnétiques destinées à être utilisées selon la revendication 7, **caractérisées en ce que** les tailles des parties magnétiques synthétisées sont comprises entre 1 et 5 µm.

9. Microparticules magnétiques destinées à être utilisées selon la revendication 8, **caractérisées en ce que** la surface des particules magnétiques synthétisées est premièrement modifiée avec de l'acide polyacrylique.

10. Microparticules magnétiques destinées à être utilisées selon la revendication 9, **caractérisées en ce que** ladite procédure de modification avec l'acide polyacrylique est constituée des étapes de processus ;
• d'ajout de particules magnétiques à une solution d'acide polyacrylique qui est dissous dans l'eau et leur mélange,
• après mélange, leur séparation par l'intermédiaire d'un aimant et leur lavage.

11. Microparticules magnétiques destinées à être utilisées selon la revendication 10, **caractérisées en ce que** l'utilisation d'un procédé de couche par couche sur les particules fonctionnelles d'acide acquises comprend les étapes de procédé comprenant ;
• premièrement, leur ajout à une solution de collagène,
• ensuite, leur ajout à une solution de glycosaminoglycane et leurs mélanges pendant 3 heures dans chaque solution.

12. Microparticules magnétiques destinées à être utilisées selon la revendication 11, **caractérisées en ce qu'**elles sont lavées avec de l'eau à chaque fois, en utilisant une séparation au moyen d'un procédé magnétique après ladite procédure de mélange.

13. Kit (1) destiné à être utilisé dans la réparation d'une couche de glycosaminoglycane qui est endommagée dans la vessie à cause d'une cystite interstitielle, **caractérisé en ce qu'**il comprend
• des microparticules magnétiques revêtues de glycosaminoglycane comme mentionné dans la revendication 1 ou 12,
• une bande magnétique sous-cutanée (20) qui interagit avec les microparticules magnétiques et qui libère le glycosaminoglycane,
moyennant quoi ladite bande magnétique sous-cutanée doit être placée dans la zone sus-pubienne de sorte que ledit glycosaminoglycane soit conservé dans la vessie (10) et reste longtemps dans la vessie (10).

14. Kit (1) destiné à être utilisé selon la revendication 13, **caractérisé en ce que** ladite bande magnétique (20) doit être attachée à la zone sus-pubienne par l'intermédiaire d'une suture (21).

15. Kit (1) destiné à être utilisé selon la revendication 13, **caractérisé en ce que** lesdites microparticules magnétiques revêtues de glycosaminoglycane doivent être introduites dans la vessie (10) par l'intermédiaire d'une seringue (40).
